Europäisches Patentamt

European Patent Office

Office européen des brevets

(19)

(11) Publication number: **0 208 507 B1**

# EUROPEAN PATENT SPECIFICATION

(45) Date of publication of patent specification: **29.04.92**   (51) Int. Cl.5: **A61K 39/00**

(21) Application number: **86305137.1**

(22) Date of filing: **02.07.86**

(54) **Tick vaccine.**

(30) Priority: **03.07.85 AU 1310/85**

(43) Date of publication of application:
**14.01.87 Bulletin 87/03**

(45) Publication of the grant of the patent:
**29.04.92 Bulletin 92/18**

(84) Designated Contracting States:
**AT BE CH DE FR GB IT LI LU NL SE**

(56) References cited:

**NATURE, vol. 280., August 9, 1979, pages
491-493, J.R. ALLEN et al.: "Immunisation of
guinea pigs and cattle against ticks"**

**COMMONWEALTH AGRICULTURAL BUREAU
REF. 86176961, CAB 860533005 J.A.Y. JOHN-
STON et al.: "Immunization of cattle against
Boophilus microplus using estracts derived
from adult female ticks: effects of induced
immunity on tick populations" & INTERNA-
TIONAL JOURNAL FOR PARASITOLOGY, vol.
16, no. 1, 1986, p. 27-34**

**CHEMICAL ABSTRACTS, vol. 85, no. 23, De-
zember 6, 1976, page 363, abstract 175339z**

Columbus, Ohio, US P. WILLADSEN et al.
**"Isolation and partial characterization of an
antigen from the cattle tick, Boophilus
microplus" & IMMUNOCHEMISTRY, 1976,
13(7), 591-7**

(73) Proprietor: **PITMAN-MOORE AUSTRALIA
LIMITED
71, Epping Road
North Ryde New South Wales 2113(AU)**

(72) Inventor: **Opdebeeck, Johanna Patricia
60, Gerler Street
Bardon Brisbane 4065 Oueensland(AU)**

(74) Representative: **Matthews, Derek Peter et al
Frank B. Dehn & Co. Imperial House 15-19
Kingsway
London WC2B 6UZ(GB)**

EP 0 208 507 B1

**Description**

This invention relates to antigenic compositions and in particular to vaccines for the control of tick infestations on animals.

In many countries the control of tick infestations on animals, particularly cattle, is a problem of considerable economic significance. Even with mild infestation there is a loss in meat and milk production and hide damage, and with heavier infestations the ticks must be removed to avoid death of the cattle. Ticks, are also disease carriers of, for example, Babesia parasites which lead to outbreaks of "tick fever".

The traditional method of control has involved the use of sprays or dipping vats with various chemicals, such as DDT, chlorinated hydrocarbons, carbamates, organophosphates, pyrethroids, and amidines. Most of these chemicals have had a limited period of commercial use because various strains of ticks emerged which were resistant to them. In some cases it was possible to extend the usefulness of a particular chemical by increasing the concentration of the chemical in the spraying or dipping fluid, but this increased the cost and frequently toxic symptoms were observed in the cattle. In general the dipping or spraying treatment needs to be repeated every three to four weeks and there are additional high labour costs involved in mustering the animals and moving them to and from the dipping or spraying sites.

There are further difficulties associated with the use of these chemicals. Where the chemicals are used in a concrete dipping vat set in the ground there is considerable contamination of the dip contents by urine and faeces from the cattle leading to decomposition and hence additional loss of the chemicals, and necessitating regular chemical analyses to monitor the level of the active ingredient. The dips need to be cleaned periodically and this leads to exposure of workers to potentially hazardous chemicals. Furthermore, the disposal of dipping fluids has to be done with care to avoid environmental damage.

A further difficulty in the use of chemicals lies in the increasingly difficult task of finding new effective chemicals to combat each new resistant tick strain as it emerges. Part of this difficulty is compounded because it is desirable that the new chemical leaves minimal residues in the meat and milk of the host animal, and that such residues that are left are not harmful to consumers.

Thus for many decades there has been an urgent need for alternative safe and cost-effective means of controlling tick infestations to cattle. Management approaches such as pasture spelling have limited effectiveness and in most cases the required areas of land are simply not available. Attempts to breed tick-resistant cattle have continued for many years with little impact on the problem. Eradication campaigns have been tried and have given limited success in some areas, albeit at high cost, but are impracticable in view of the very large areas which may be involved.

We have now discovered an effective method of tick control which does not require the use of hazardous chemicals, and since a single treatment is effective for extended periods the need for regular mustering is eliminated. This method depends on the capacity of the animal's immune systems to prevent tick survival or development on the animal.

Use of resistant cattle for the control of cattle tick has been suggested as a supplement to tick control by chemicals. The stimulus of tick infestation is normally required before resistance is manifested; this resistance is "acquired" and is presumed to be immunologically mediated. However the degree of resistance which an individual bovine can develop is a heritable characteristic which varies widely according to breed. Some breeds, for example Zebu and part Zebu crossbreeds develop high levels of resistance while others, such as most British breeds, remain largely susceptible. Despite extensive selective breeding programs, however, this natural resistance has not provided a satisfactory alternative or supplement to the chemical control of ticks.

The possibility of developing a tick vaccine has been suggested previously. Whole tick homogenates, salivary antigens, gut-associated antigens and certain purified proteins or enzymes have been used by various workers (See Allen and Humphries, Nature (1979) 280, 491-493) and some have produced levels of immunity which are significant but still inadequate by comparison with chemical methods of control. Various explanations for this limited success have been proposed; for example, that vaccines cannot be expected to produce a greater resistance than the naturally acquired resistance that is characteristic of the breed concerned, that whole tick homogenates include immuno-suppressive components, and that purified antigens are inadequate and require synergy with other antigens.

Australian patent specification 45936/85 discloses an attempt to prepare tick antigens by purification of crude whole tick extracts. However, the reported level of isolation of useful antigens is low.

The present invention for vaccinating cattle against cattle tick confers a high long-lasting level of resistance on naive cattle of breeds which characteristically develop only a small degree of natural resistance. The efficacy of the vaccine is such as to offer a very satisfactory alternative to chemical control, as well as the advantage of very infrequent treatments.

2

EP 0 208 507 B1

Accordingly we provide an antigenic composition wherein the antigenic material is the synganglion of a tick.

It is also found that antigenic material derived from the synganglion (i.e. the central nervous system of the tick) extends and improves the protection given by other antigens e.g. those prepared from the tick gut alone. While we do not wish to be limited by discussion of the possible underlying basis for our invention, it is possible that there are materials in the whole body of the tick that interfere with the generation of an effective immune response otherwise stimulated by the antigens of the synganglion. Thus, in a further embodiment of our invention we provide a mixture wherein the antigens are derived from both the synganglion and the gut of the tick.

In another aspect of our invention, we provide a vaccine for the protection of warm-blooded animals against ticks prepared from antigens which are obtained by dissection of synganglia or other nerve tissues from adult ticks. The dissected tissues may he homogenized, sonicated and centrifuged. The resulting mixture of antigens comprises both water-soluble and particulate materials. We have found the particulate fraction to be more antigenic.

In a preferred aspect of our invention, the antigenic materials are obtained from larval ticks. The advantage of this method lies in the higher proportion of synganglion in the body-weight of the larval tick compared to that of the nymph or adult stages of the ticks. The larval ticks may be extracted by grinding or homogenizing or otherwise disrupting the ticks, followed by extraction with suitable aqueous media, fractionation by suitable methods of affinity chromatography, gel permeation chromatography, ion-exchange chromatography, hydrophobic gel chromatography, electrophoresis, electrofocussing, selective precipitation, and concentration to provide the final antigenic materials enriched in the antigenic components.

In a further embodiment of our invention, antigenic materials are obtained by disruption of developing tick eggs at the stage when the materials are first synthesized in the growing embryo.

The antigenic materials may also be obtained by cloning and expression of tick genetic material in suitable host organisms, including bacteria, yeasts and other microorganisms and in cultured mammalian cells.

In another embodiment of our invention, antigenic materials characteristic of the synganglion are obtained by in vitro culture of tick cells or tick cells hybridized with other animal cells.

In a further aspect of our invention, antigenic materials are obtained by affinity chromatography or immune precipitation or otherwise using serum antibodies of warm-blooded animals successfully immunized with tick antigenic material, or using monoclonal antibodies which bind specifically to tick antigenic material.

In a further embodiment of our invention antigenic materials are obtained by binding to immobilized ligands which are related to known functional molecules of the nervous system, for example neutrotransmitter molecules, or growth factors characteristic of nervous systems, or their derivatives respectively.

The antigenic materials are combined with one or more suitable adjuvants known to those skilled in the art, for example saponins (or derivative or related material), "ISCOMS", muramyl dipeptide, trehalose dimycollate, Freund's complete adjuvant, Freund's incomplete adjuvant, other water-in-oil emulsions, double emulsions, dextran, diethylaminoethyl-dextran, potassium alum, aluminium phosphate, aluminium hydroxide, bentonite, zymosan, polyelectrolytes, retinol, calcium phosphate, protamine, sarcosine, glycerol, sorbitol, propylene glycol, carboxyvinyl polymers available under the registered trade mark "Carbopol", fixed oils and synthetic esters of higher fatty acids Saponins have been found to be particularly effective adjuvants.

The antigenic material may be treated with a suitable preservative including for example phenol, "Phenonip", formaldehyde, propylene glycol, glycerol, esters of p-hydroxybenzoic acid, benzoic acid and its sodium salt, hexachlorophene, quaternary germicides, sodium azide and thiomersal used as such or in the form in which it is available under the registered trade mark "Merthiolate".

The antigenic material may be sterilized prior to or after formulation as appropriate, by filtration, irradiation or chemical treatment.

The antigenic material may also be coupled chemically or physically to a suitable carrier, such as latex particles, or immunogenic macro-molecules, or agarose beads or suchlike, or it may be formulated as antigen complexed with bovine or other antibodies.

The antigenic material may also be treated with a suitable inhibitor, modifier, crosslinker or denaturant, or by heat or irradiation, in such a way as to conserve or enhance its immunogenic efficacy.

The antigenic material may also be used in combination with other therapeutic agents such as anthelmintics, for example levamisole or its therapeutically acceptable salts, flukicides and other vaccines, for example clostridial vaccines.

3

The antigenic material may also be used in combination with immunostimulatory agents such as levamisole, bestatin, tuftsin, lectine, bacterial lipopolysaccharides, polynucleotides, tilorone, lentinan, isoprinosine, lysolecithin, and the like, or with immunoregulatory hormones such as interleukins, leukotrienes and the like or with compositions of the nature of transfer factor.

The antigenic material may be freeze-dried or otherwise dried or concentrated, and reconstituted just prior to use by addition of a suitable liquid medium, for example a sterile saline solution, optionally with the addition of one or more adjuvants or other additive materials alluded to above.

The antigenic material formulated by any of the means described is henceforth termed the vaccine.

The amount of the vaccine administered to the animal will depend on the bodyweight of the animal and the relative activity of the particular preparation of antigenic material. Preferably the vaccine is formulated in such a manner that sufficient antigen to protect the animal is contained in a volume of from 1 to 10 ml of vaccine, and more preferably in 1 to 5 ml of vaccine. The amount of antigenic material required is very small and typically samples of antigenic material containing less than 2 mg of protein (preferably 10-1000 micrograms for cattle, particularly 50-500 micrograms) are sufficient for effective immunization of the animals against tick infestation. For other animals e.g. dogs or poultry correspondingly smaller doses would be used.

The preferred mode of administration of the vaccine is parenteral. The term "parenteral" is used herein to mean intravenous, intramuscular, intradermal, and subcutaneous injection. The administration is most conveniently carried out by intramuscular injection. The conventional injection guns used for other therapeutic applications with cattle may conveniently be used.

The vaccine may be used for the control or eradication of, or protection from infestation by, Argasid and Ixodid ticks, including species such as Boophilus spp., Rhipicephalus spp., Ixodes spp., Hyalomma spp., Amblyomma spp., Dermacentor spp., and Haemaphysalis spp.

The vaccine may also be administered in the form of a depot or slow-release device or controlled-release device implanted in or attached to any part of the animal.

The novel method for the control of ticks provided by the invention has a number of advantages. As mentioned hereinbefore the method of the invention avoids the use of hazardous chemicals with the attendant difficulties of maintaining and cleaning dipping vats and spraying equipment, but there are many additional advantages.

These additional advantages include the ease of combining treatment for control of ticks with other therapeutic treatments, the simple labour-saving method required for administration, elimination of possibly toxic insecticides being added to the environment, prevention of the development of resistant strains of ticks, and long periods of protection. This period of protection is advantageous both in reducing the need for regular treatments and because animals are protected at the start of each new tick season when the number of ticks in the pastures is low and likely to go unnoticed. In most situations an annual vaccination will be sufficient to confer year-round protection of animals from tick attack.

The effect of the vaccine is not only to reduce the yield of ticks from infested cattle, but also to reduce the viability and fertility of the surviving ticks so that few eggs, generally of low viability, are produced. This phenomenon accentuates the effect of widespread vaccination in reducing contamination of pasture with ticks.

In the case of the cattle tick, Boophilus microplus, which has a single host in its complete life cycle, the extended period of protection will also have the effect of steadily reducing the tick levels in the pastures and this effect will accumulate from year to year. A similar effect will occur with multihost ticks.

The invention is now illustrated by, but by no means limited to, the following Examples.

EXAMPLE I

Two Hereford calves, without prior exposure to ticks, were vaccinated with antigen preparations prepared by homogenizing guts and synganglia obtained by dissection of adult cattle tick (Boophilus microplus). Particulate and water-soluble fractions were obtained by centrifugation. The particulate fraction was administered as a suspension with saponin (Quil A), the soluble fraction was emulsified with Freund's incomplete adjuvant, and the two were injected subcutaneously at different sites. The vaccines were administered three times, on days 0, 14 and 45 of the trial. The doses included 500 micrograms of soluble antigen protein on each occasion, and 350, 100 and 100 micrograms of particulate antigen protein on the three days respectively.

A similar pair of calves was tested at the same time with the adjuvants only, and a third pair was untreated.

The calves were challenged on days 62, 69, 76, 83 and 90 with 20,000 larval ticks on each occasion.

All the adult ticks which dropped from the calves were counted and incubated until oviposition was complete. Ticks which were abnormal in size or appearance were also noted and incubated separately.

The table gives the cumulative totals for normal and abnormal ticks and for the eggs produced. The vaccinated calves yielded the following reductions relative to the control calves:

Total ticks                                                     - reduced by 85%
Normal ticks                                                  - reduced by 96%
Total eggs                                                    - reduced by 98%
Mean weight of normal ticks                           - reduced by 50%
Mean weight of eggs produced by normal ticks   - reduced by 66%

TABLE 1

|  | Total Tick | Normal Tick | Eggs(g) |
|---|---|---|---|
| Adjuvant controls | 30,200 | 25,120 | 2,239 |
| Vaccinated gut & synganglion | 4,900 | 910 | 50 |

EXAMPLE II

Preparation of tick vaccine, using affinity chromatography to extract protective antigens from larval tick komopenatet

Summary

A) Production of Antiserum

. Guts and synganglia are dissected from adult ticks
. Vaccines are prepared from these organs and administered to cattle.
. The cattle are infested with ticks and shown to be highly immune.

B) Preparation of Affinity Column

. Serum is prepared from the immune cattle from (A), antibodies are extracted from the serum, and bonded to chromatographic support.

C) Chromatographic Extraction of Antigens

. Larval ticks are homogenised and separated into soluble and particulate fractions
. Both fractions are applied to the immobilised serum antibodies from (B)
. Antigens which bind to the immobilized antibodies are eluted and formulated as a vaccine

D) Vaccination of Cattle

. cattle vaccinated with the antigens obtained from (C) are substantially immune to tick infestation, whereas whole larval tick homogenates are ineffective in vaccination.

A) PRODUCTION OF ANTISERUM

Collection of tick organs. Female ticks (Boophilus microplus) were collected manually from 2 Hereford cattle following the moult to young adult (day 14-17 of the life cycle). The ticks were embedded in wax and the following organs were dissected out:
1. Synganglion
2. Gut
Organs were snap frozen on dry ice and stored at -20°C until used.

Preparation of antigens

(1) Guts were suspended in 25 mM Tris buffer containing 132.5 mM NaCl and 1 mM $Na_2$ EDTA, pH 7.2, (antigen buffer) and were homogenized in a manual Dowse homogenizer. The homogenate are sonicated using 30-60 second bursts for a total of 10 minutes. Sonication was followed by centrifugation at 600 g for 10 minutes. Pellets were rehomogenized in an aliquot of supernatant and sonicated as above for a further 5 minutes. The sonicated preparations were pooled and centrifuged at 15,000 g for 20 minutes and the cell pellet was discarded. The supernatant was then subjected to centrifugation at 100,000 g for 1 h. The supernatant thus obtained was retained (soluble protein fraction) and the membrane pellet resuspended by homogenization in antigen buffer. Soluble and membrane fractions were stored at -20°C until used. All steps described above were carried out in ice baths.

(2) Synganglia were forced through a fine mesh using a pestle. The cell suspension was collected in antigen buffer and frozen at -20°C until used.

Vaccination preparation

Soluble protein fractions and cell suspensions were emulsified in an equal volume of Freunds incomplete adjuvant. Membrane protein fractions were suspended in antigen buffer containing 1 mg saponin (Quil A) per ml. All vaccine preparations were made up on the day of immunization.

Administration of vaccines

Vaccine preparations were administered intramuscularly using in 18 g needle as set out in Table 2. Membrane preparations were inoculated in the middle third of the right side of the neck, soluble protein preparations were administered in the middle third of the left neck and cell suspensions were inoculated into the middle third of the semimembranosus and semitendinous muscles of the left hind leg. Control animals were either uninoculated or received adjuvant plus saline. Vaccines were administered on day 0, 11 and 12 of the experiment.

Blood sampling procedures

Animals were bled from the jugular vein at regular intervals; serum was collected by centrifugation and stored at -20°C.

Challenge trial

The cattle were exposed to tick larvae, (ten days old;

TABLE 2 Vaccine program concentrations and mode of presentation of antigen

| Vaccine group | Time (days) | | | | | | # of animals |
| --- | --- | --- | --- | --- | --- | --- | --- |
| | 0 | | 14 | | 42 | | |
| | Membrane | Soluble | Membrane | Soluble | Membrane | Soluble | |
| 1. Gut plus synganglia | 350Δ *500 | 500 | 100 75 | 500 | 50 50 | 500 | 2 |
| 2. Uninoculated controls | - | - | - | - | - | - | 2 |
| 3. Adjuvant only controls | - | - | - | - | - | - | 2 |

* Synganglia were administered as single cell suspensions
Δ Protein concentrations are in micrograms

20,000 per infestation) which were placed on the cattle on five occasions at seven-day intervals. All normal and damaged ticks were collected and counted and their production of eggs and larvae determined.

## RESULTS

### Table 3

| | TICK NUMBERS | | | EGG WT. |
|---|---|---|---|---|
| | normal | damaged | total | grams |
| Uninoculated controls | 17380 | 1599 | 18988 | 2276 |
| Adjuvant controls | 17798 | 987 | 18785 | 1787 |
| Synganglion – gut | 820 | 2240 | 8060 | 54 |

Percent protection by vaccine:–

| | |
|---|---|
| Normal Ticks | 95% |
| Total Ticks | 84% |
| Eggs | 97% |

## B) PREPARATION OF AFFINITY COLUMN

Sera from the immune cattle were precipitated with 50% ammonium sulphate for 4 hours at room temperature. Following extensive dialysis against affinity coupling buffer; 0.1 M $NaHCO_3$, 0.5 M NaCl, P.H. 8.5 100 mgs of immunoglobulins (Igs) were coupled to 10 ml of cyanogen bromide activated sepharose 48 gel. Remaining reactive sites were blocked with 0.2 M glycine buffer p.H. 8.0. Following coupling and washing, gels were packed in 15 ml columns. Normal Igs from control animals, subsequently proven to be highly susceptible to ticks, were also coupled to gel and columns were prepared as previously. In addition, activated Sepharose 4B gel was blocked by 0.2M glycine buffer, p.H. 8.0, and used to control non-specific absorbtion of proteins to gel.

## C) CHROMATOGRAPHIC EXTRACTION OF ANTIGENS

Large quantities of eggs (10 gms - 100 gms,) collected over a 7-day laying period from batches of female ticks, were hatched in containers. The 10 day old larvae were frozen for 1 hour, separated from the egg debris, and extracted as described for gut vaccine (see A)

Antigens were dialyzed in affinity buffer (PBS p.H. 7.4) and 50 mgm protein was applied to the loaded affinity column. Antigen was incubated on the column for 30 minutes, then the column was extensively washed with PBS p.H. 7.4 at 20 ml/hr$^{-1}$ until baseline O.D. readings at 280 nm were re-established. Glycine buffer 0.1 M, p.H. 2.8 was applied and fractions were collected until baseline O.D. readings were again re-established. The column was stored in Tris buffer containing sodium azide and regenerated the following day using high and low pH buffer cycles.

Particulate and soluble fractions from larval tick homogenates were both separately chromatographed in this way. The particulate fraction was first treated with Triton X-100 but was not centrifuged. Both soluble and particulate materials were subsequently specifically eluted together.

D) VACCINATION OF CATTLE

Affinity chromatographed larval antigens were formulated as for gut antigens (see (A) above) and administered to cattle on days 0, 28 and 42 to a total of 500 micrograms of protein per animal.

Table 4

| RESULTS | | |
|---|---|---|
| Vaccine Group | Percent Protection | |
| | (Total Ticks) | (Eggs) |
| Whole larval homogenate (soluble plus particulate) | 16% | 25% |
| Affinity purified fraction from whole larval homogenate (soluble plus particulate) | 65% | 84% |

EXAMPLE III

Efficacy of Vaccines based on dissected tick organs

Method

. Guts and synganglia are dissected from adult ticks as described in Example II (A).
. Vaccines are prepared and administered to cattle, either gut preparations (soluble and particulate) or synganglion (cell suspension) or both together, as described in Example II (D) and Table 5.
. Cattle are infested with ticks and immunity assessed as described in Example II(A).

Results

(a) Percent Protection against three successive weekly challenges commenced on day 56 of trial:-

| | Total Ticks | Eggs |
|---|---|---|
| Synganglion plus gut | 79% | 91% |
| gut alone | 88% | 96% |

(b) Percent Protection against natural infestations at approximately 6 months after first vaccination:-

| | |
|---|---|
| Synganglion plus gut | 65% (ticks) |
| Gut alone | [-20%] |

Thus whilst the protection given by synganglion plus gut is comparable to gut alone at 56 days, the long term protection at 6 months is markedly superior.

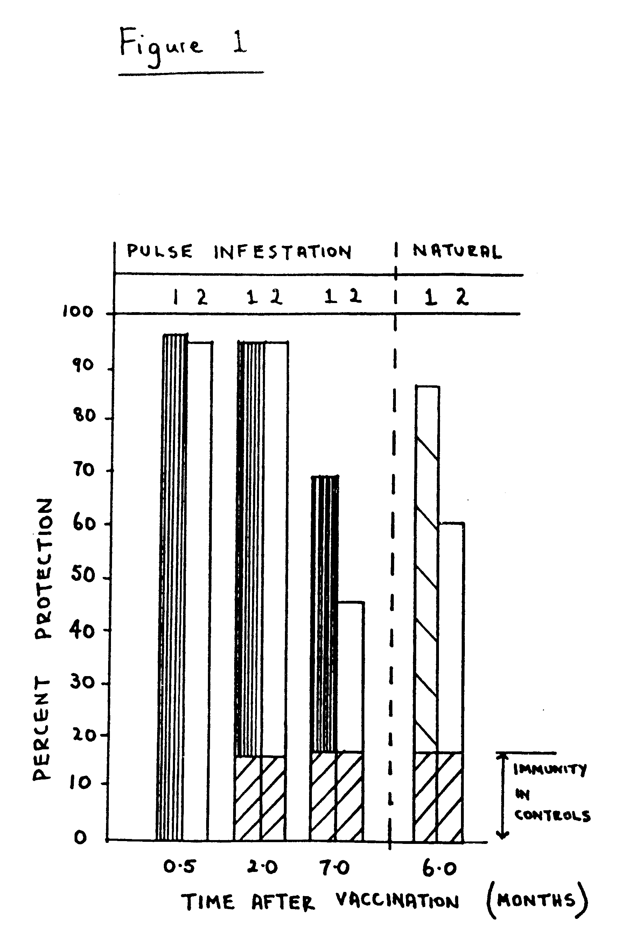

These results are shown in more detail in Figure 1.

Figure 1 shows the cumulative drop of ticks from 2 groups of vaccinated cattle compared to the mean cumulative tcks dropped by controls. Group 1 was vaccinated with antigen derived from both synganglion and gut. Group 2 was vaccinated with antigen from gut alone. The level of immunity (percent protection) was determined by challenging the two groups and the controls with pulse infestations of 20,000 B microplus larvae; and also by natural infestation in tick-infested pasture.

The results show enhanced long-term protection in Group 1 (synganglion and gut) relative to Group 2 (gut alone) relative both to pulse and natural tick challenges. There is also a degree of natural immunity in controls.

TABLE 5   Vaccines administered to animals in Example III

| Animal | Antigen | | | | | | | | |
|---|---|---|---|---|---|---|---|---|---|
| | Gut membrane | | | Gut supernatant | | | Synganglion | | |
| | 1 | 2 | 3 | 1 | 2 | 3 | 1 | 2 | 3 |
| 27, 28 | 350 | 100 | 50 | 500 | 500 | 500 | - | - | - |
| 29, 30 | 350 | 100 | 50 | 500 | 500 | 500 | - | - | - |
| 31, 32 | 350 | 100 | 50 | 500 | 500 | 500 | 500 | 75 | 50 |
| 33, 34, 35 | | | | | | | | | |
| 36, 37 | - | - | - | - | - | - | - | - | - |
| 38, 39 | | | | | | | | | |
| 40 (Controls) | | | | | | | | | |

## Claims

1. An antigenic composition wherein the antigenic material is derived from the synganglion of a tick.

2. A composition according to claim 1 wherein the synganglion-containing antigenic material is derived from tick larvae.

3. A composition according to claim 1 or 2 which further comprises antigenic material derived from the gut of the tick.

4. A composition according to any preceding claim wherein the antigenic material has been purified by passing over an affinity chromatography substrate having antibodies to tick antigenic material bound thereto.

5. A composition according to any preceding claim wherein the tick is Boophilus microplus.

6. A composition according to any preceding claim which further comprises saponin as an adjuvant.

7. A process for the production of purified antigenic material wherein the antigenic material is derived from the synganglia of ticks, which process comprises:
(i) preparation of an affinity chromatography substrate having bound thereto antibodies to tick antigenic material;
(ii) passing crude tick extract over the substrate, so as to adsorb tick antigens on to the substrate; and
(iii) eluting purified antigenic material from the substrate.

8. A process according to claim 7 wherein the crude tick extract is derived from tick larvae.

9. A process for the production of purified antigenic material wherein the antigenic material is derived from the synganglia of ticks and guts of tick larvae, which process comprises:
(i) preparation of an affinity chromatography substrate having bound thereto antibodies to tick antigenic material;
(ii) passing crude tick extract over the substrate, so as to adsorb tick antigens on to the substrate; and
(iii) eluting purified antigenic material from the substrate.

10. A process according to claim 7, 8 or 9 wherein the antibodies bound to the affinity chromatography substrate are derived from blood serum from an animal successfully immunised against tick.

11. A process according to claim 7, 8 or 9 wherein the antibodies bound to the affinity chromatography substrate are monoclonal antibodies produced against tick antigenic material.

12. The use of antigenic material derived from the synganglia of ticks for the manufacture of a medicament for the control of tick infestation of a warm-blooded animal.

**Revendications**

1. Composition antigénique, dans laquelle le matériel antigénique provient de la masse ganglionnaire d'une tique.

2. Composition selon la revendication 1, dans laquelle le matériel antigénique contenant la masse ganglionnaire provient de larves de tiques.

3. Composition selon la revendication 1 ou 2, qui comprend en outre du matériel antigénique provenant de l'intestin de la tique.

4. Composition selon l'une quelconque des revendications précédentes, dans laquelle le matériel antigénique a été purifié par passage sur un substrat de chromatographie d'affinité auquel sont fixés des anticorps dirigés contre le matériel antigénique de la tique.

5. Composition selon l'une quelconque des revendications précédentes, dans laquelle la tique est Boophilus microplus.

6. Composition selon l'une quelconque des revendications précédentes, qui comprend en outre de la saponine comme adjuvant.

EP 0 208 507 B1

**7.** Procédé de production de matériel antigénique purifié, dans lequel le matériel antigénique provient des masses ganglionnaires de tiques, lequel procédé comprend :

(i) la préparation d'un substrat de chromatographie d'affinité auquel sont fixés des anticorps dirigés contre le matériel antigénique de la tique ;

(ii) le passage de l'extrait brut de tique sur le substrat, de façon à adsorber les antigènes de tique sur le substrat ; et

(iii) l'élution du matériel antigénique purifié du substrat.

**8.** Procédé selon la revendication 7, dans lequel l'extrait brut de tique provient de larves de tiques.

**9.** Procédé de production de matériel antigénique purifié, dans lequel le matériel antigénique provient des masses ganglionnaires de tiques et des intestins de larves de tiques, lequel procédé comprend :

(i) la préparation d'un substrat de chromatographie d'affinité auquel sont fixés des anticorps dirigés contre le matériel antigénique de la tique ;

(ii) le passage de l'extrait brut de tique sur le substrat, de façon à adsorber les antigènes de tique sur le substrat ; et

(iii) l'élution du matériel antigénique purifié du substrat.

**10.** Procédé selon la revendication 7, 8 ou 9, dans lequel les anticorps fixés au substrat de chromatographie d'affinité proviennent du sérum sanguin d'un animal immunisé avec succès contre la tique.

**11.** Procédé selon la revendication 7, 8 ou 9, dans lequel les anticorps fixés au substrat de chromatographie d'affinité sont des anticorps monoclonaux produits contre le matériel antigénique de la tique.

**12.** Emploi de matériel antigénique provenant des masses ganglionnaires de tiques pour la préparation d'un médicament destiné au contrôle de l'infestation d'un animal à sang chaud par des tiques.

**Patentansprüche**

**1.** Antigene Zusammensetzung, bei der das antigene Material von dem Synganglion einer Zecke abgeleitet ist.

**2.** Zusammensetzung nach Anspruch 1, bei der das synganglionhaltige antigene Material von Zeckenlarven abgeleitet ist.

**3.** Zusammensetzung nach Anspruch 1 oder 2, die weiterhin von dem Darm der Zecke abgeleitetes antigenes Material enthält.

**4.** Zusammensetzung nach jedem der vorhergehenden Ansprüche, bei der das antigene Material durch Leiten über ein Affinitätschromatografie-Substrat mit an dieses gebundenen Antikörpern gegen antigenes Zeckenmaterial gereinigt worden ist.

**5.** Zusammensetzung nach jedem der vorhergehenden Ansprüche, bei der die Zecke Boophilus microplus ist.

**6.** Zusammensetzung nach jedem der vorhergehenden Ansprüche, die weiterhin Saponin als Adjuvans enthält.

**7.** Verfahren zur Herstellung von gereinigtem antigenen Material, bei dem das antigene Material von den Synganglia von Zecken abgeleitet ist, mit den Merkmalen:

(i) Herstellung eines Affinitätschromatografie-Substrats mit an dieses gebundenen Antikörpern gegen antigenes Zeckenmaterial;

(ii) Leiten eines rohen Zeckenextrakts über das Substrat zur Adsorption von Zeckenantigenen an dem Substrat; und

(iii) Elution von gereinigtem antigenen Material von dem Substrat.

**8.** Verfahren nach Anspruch 7, bei dem der rohe Zeckenextrakt von Zeckenlarven abgeleitet ist.

12

**9.** Verfahren zur Herstellung von gereinigtem antigenen Material, bei dem das antigene Material von den Synganglia von Zecken und den Därmen von Zeckenlarven abgeleitet ist, mit den Merkmalen:

(i) Herstellung eines Affinitätschromatografie-Substrats mit an dieses gebundenen Antikörpern gegen antigenes Zeckenmaterial;

(ii) Leiten eines rohen Zeckenextrakts über das Substrat zur Adsorption von Zeckenantigenen an dem Substrat; und

(iii) Elution von gereinigtem antigenen Material von dem Substrat.

**10.** Verfahren nach Anspruch 7, 8 oder 9, bei dem die an das Affinitätschromatografie-Substrat gebundenen Antikörper aus dem Blutserum eines erfolgreich gegen Zecken immunisierten Lebewesens abgeleitet sind.

**11.** Verfahren nach Anspruch 7, 8 oder 9, bei dem die an das Affinitätschromatografie-Substrat gebundenen Antikörper gegen antigenes Zeckenmaterial gebildete monoklonale Antikörper sind.

**12.** Verwendung von von den Synganglia von Zecken abgeleitetem antigenen Material für die Herstellung eines Medikamentes zur Kontrollierung des Zeckenbefalls bei einem warmblütigen Lebewesen.

# Figure 1